# EUROPEAN PATENT APPLICATION

(11) **EP 2 298 198 A1**
(43) Date of publication of application: **23.03.2011**
(21) Application number: 09773467.7
(22) Date of filing: 30.06.2009
(51) Int. Cl.: A61B 17/3211, A61F 9/007, B26B 9/00

(54) **MEDICAL EDGED INSTRUMENT AND METHOD OF COATING MEDICAL EDGED INSTRUMENT**

(30) Priority: 30.06.2008 JP 2008170024
(71) Applicant: Mani, Inc., Tochigi 321-3231 (JP)
(72) Inventor: YAMAGUCHI, Akio, Utsunomiya-shi Tochigi 321-3231 (JP); SAITO, Masahiko, Utsunomiya-shi Tochigi 321-3231 (JP); OGANE, Kaoru, Utsunomiya-shi Tochigi 321-3231 (JP)
(74) Representative: Patentanwälte Westphal, Mussgnug & Partner
(86) International application number: PCT/JP2009/061951
(87) International publication number: WO 2010/001889

(57) **Abstract**

Provided is a medical bladed device reduced in piercing resistance by coating with a silicone. Also provided is a coating method by which a satisfactory silicone coating is formed on a medical bladed device. A medical bladed device (knife (A)) having a cutting portion (1) with an edge (3) on the periphery thereof and a shank (2) is subjected to coating to coat the cutting portion (1) including the edge (3) with a silicone having a thickness of from 25 nanometers to 5 micrometers. While holding the medical bladed device, with the cutting portion (1) lower and the shank (2) upper, the cutting portion is immersed in a silicone solution so that the angle between the upper or lower side of the cutting portion and the solution surface (21) of the silicone solution is 0-90 degrees. Thereafter, the medical bladed device is raised at a rate of 500 mm/min or less while keeping the angle in that range to separate the cutting portion from the silicone solution. The cutting portion is then dried at a temperature in range of 100-250 °C for 30 minutes or longer. The silicone concentration is 0.2 -15 % by weight, and the thickness of the silicone film is regulated by conducting the coating operation once or repeatedly.

## Description

### Technical Field

The present invention relates to a medical bladed device that is improved in piercing ability when used for an operation and a coating method used in manufacturing the medical bladed device.

### Background Art

One example of the medical bladed device is an ophthalmic knife used in ophthalmic surgery. The ophthalmic knife is used for incising a cornea or a sclera that constitute an eyeball and realizes formation of an initial incision wound in a cornea or formation of an incision wound having good self-closing property. It has been alleged that the resistance acting when incising a cornea using an ophthalmic knife is low and doctors are not affected thereby. However, a higher resistance than that acting when incising a cornea is produced when incising a sclera, and there has been a demand for reducing such resistance.

Meanwhile, the piercing resistance acting when inserting a medical suture needle into a body tissue also fatigues doctors, and there has therefore been a demand to reduce such piercing resistance. In order to meet such demands, silicone coating is applied on the surface of a needle (for example, see Patent Documents 1 to 3).

For example, the invention disclosed in Patent Document 1 is a suture needle that is made of an austenitic stainless steel material having a baked silicone coating layer on the surface thereof, and can exert an effect that is capable of maintaining good piercing property even when an increased number of piercing operations are performed. The invention disclosed in Patent Document 2 is a square suture needle having patterns formed in faces constituting a cutting portion of a square suture needle in the direction substantially perpendicular to the center axis and in the direction along the center axis, and a silicone coating layer. According to this invention, the silicone coating layer is formed over the entire uneven surface, thereby producing an effect that the resistance acting when the suture needle pierces a body tissue is reduced. Further, the invention disclosed in Patent Document 3 is a suture needle having a tip pickled and coated with silicone, thereby producing a lubricating effect due to silicone when the needle is pierced.

As described above, all of Patent Documents 1 to 3 describe that the piercing resistance can be reduced by forming a silicone coating layer on the surface of a suture needle.

[Patent Document 1] Japanese Examined Patent Publication No. 6-20461 (Patent No. 1895794)
[Patent Document 2] Japanese Patent Application Laid-Open No. 5-56983 (Patent No. 3140508)
[Patent Document 3] Japanese Patent No. 2599893

### Disclosure of the Invention

### Problems to be Solved by the Invention

Since the medical bladed device, in particular the ophthalmic knife, has low resistance when forming an incision wound having good self-closing property or incising a cornea and thus has sufficiently satisfactory function, application of silicone coating has not been considered. However, as there has been a demand for reducing the resistance when incising a sclera as described above, formation of a silicone coating layer on the surface including a cutting portion is expected to be effective.

Accordingly, the inventors of the present application have conducted development of medical bladed devices applied with silicone coating, but there are still many problems to be solved.

One of such problems is the method for applying silicone coating. Specifically, a sprayingmethod is employed as a method for applying silicone coating on the surface for the suture needles disclosed in Patent Documents 1 and 2. This method is carried out by spraying a silicone solution using a spray gun while visually observing the tip portion of a suture needle. In this case, the spraying is carried out carefully so that the sprayed silicone solution does not get into an eye formed in the suture needle for binding a suture thread.

For example, if silicone gets into the eye formed in the suture needle for binding a suture thread, it is difficult to have a stable binding strength and the suture thread may escape from suture needle while suturing.

Further, when a silicone solution is sprayed on the medical bladed device using a spray gun, it is difficult to form a uniform coating layer and drop-like lumps are disadvantageously formed on the surface.

When the medical bladed device is an ophthalmic knife, it is disadvantageously difficult to apply silicone over the cutting portion. Moreover, another problem is to determine the thickness of the silicone coating layer for achieving a value of piercing resistance with which doctors are satisfied.

An object of the present invention is to provide a medical bladed device that is subjected to silicone coating and thus produces reduced piercing resistance, and a coating method that can apply excellent silicone coating to the medical bladed device.

### Means for Solving the Problems

In order to solve the aforementioned problems, a medical bladed device according to the present invention includes: a cutting portion having an edge on an outer side thereof, and a shank that is continuous from the cutting portion, wherein the cutting portion including the edge is coated with silicone in a thickness of 25 nanometers or larger and 5 micrometers or smaller.

Further, a method of coating a medical bladed device according to the present invention is a method of coating a medical bladed device having a cutting portion and a shank that is continuous from the cutting portion by immersing the medical bladed device into a silicone solution to apply silicone coating on a surface of the cutting portion, including: immersing the cutting portion into the silicone solution while keeping the cutting portion side down and the shank side up and an angle between an upper surface or a lower surface of the cutting portion and a solution surface of the silicone solution within a range of 0 degree or larger and 90 degrees or lower; thereafter raising the medical bladed device at a rate of 500 mm per minute or lower within the angle range to pull the cutting portion out from the silicone solution; and drying at a temperature range of 100°C or higher and 250°C or lower for 30 minutes or more.

Preferably, in the method of coating a medical bladed device, a thickness of a silicone film is adjusted by setting a silicone concentration to be 0.2% by weight or higher and 15% by weight or lower and by performing the immersion into silicone and the drying once or a plurality of times repeatedly.

### Effects of the Invention

With the medical bladed device according to the present invention, the piercing resistance acting when the medical bladed device is inserted into a body tissue including a sclera constituting an eyeball can be reduced by coating the cutting portion including the edge with silicone in the thickness of 25 nanometers (nm) or larger and 5 micrometers (µm) or smaller.

With the method of coating a medical bladed device according to the present invention, the cutting portion including the edge can be coated with silicone by immersing the cutting portion into the silicone solution while keeping the cutting portion side down and the shank side up and the angle between an upper surface or a lower surface of the cutting portion and a solution surface of the silicone solution within a range of 0 degree or larger and 90 degrees or lower; thereafter raising the medical bladed device at a rate of 500 mm per minute or lower to pull the cutting portion out from the silicone solution; and further drying at a temperature range of 100°C or higher and 250°C or lower for 30 minutes or more.

In particular, the thickness of the silicone film can be adjusted to a desired value by setting the silicone concentration to be 0.2% by weight or higher and 15% by weight or lower and by performing the immersion into silicone and the drying once or a plurality of times repeatedly.

### Brief Description of the Drawings

FIG. 1 is a plan view for explaining the shape of a knife according to the present embodiment.
FIG. 2 is a sectional view of the knife.
FIG. 3 shows tables showing results of a comparative experiment in which the piercing resistance acting when a pig sclera was pierced was measured.
FIG. 4 shows schematic views for explaining a coating method.
FIG. 5 shows pictures of the surfaces when the knife was raised from a silicone solution at a rate of 5 mm per minute.
FIG. 6 shows pictures of the surfaces when the knife was raised from the silicone solution at a rate of 10 mm per minute.
FIG. 7 shows pictures of the surfaces when the knife was raised from the silicone solution at a rate of 30 mm per minute.
FIG. 8 shows pictures of the surfaces when the knife was raised from the silicone solution at a rate of 50 mm per minute.
FIG. 9 shows pictures of the surfaces when the knife was raised from the silicone solution at a rate of 200 mm per minute.
FIG. 10 shows pictures of the surfaces when the knife was raised from the silicone solution at a rate of 500 mm per minute.
FIG. 11 shows pictures of the surfaces when the knife was raised from the silicone solution at a rate of 1, 000 mm per minute.

### Description of Reference Numerals

- A: knife
- B: container
- C: bridge-like continuous portion
- 1: cutting portion
- 2: shank
- 3: edge
- 4: pointed tip
- 5: line
- 6, 7, 10: slanting surface
- 8, 11: flat surface
- 21: solution surface

### Best Mode for Carrying Out the Invention

Most preferable embodiments of a medical bladed device and a method for coating the medical bladed device according to the present invention will be described below.

The shape of the medical bladed device according to the present invention is not particularly limited, and the present invention may be applied to bladed devices (knives) used for medical purposes such as a bladed device (such as a suture needle and a scalpel) for incising a tissue such as skin and muscle or a bladed device for incising a cornea and a sclera that constitute an eyeball.

The material forming the bladed device according to the present invention is not limited. However, since the bladed device is required to have high hardness when used to incise a tissue, it is possible to employ carbon steel or martensitic stainless steel, which can be expected to harden by quenching.

In the case where the bladed device is likely to rust during the course of distribution, austenitic stainless steel is preferably employed. The austenitic stainless steel cannot be expected to harden by quenching, and thus it is necessary to harden by cold plastic working. Examples of such cold working include methods such as cold wire drawing and cold forging, which are selectively employed as appropriate according to the shape and the size of the intended bladed device.

### First Embodiment

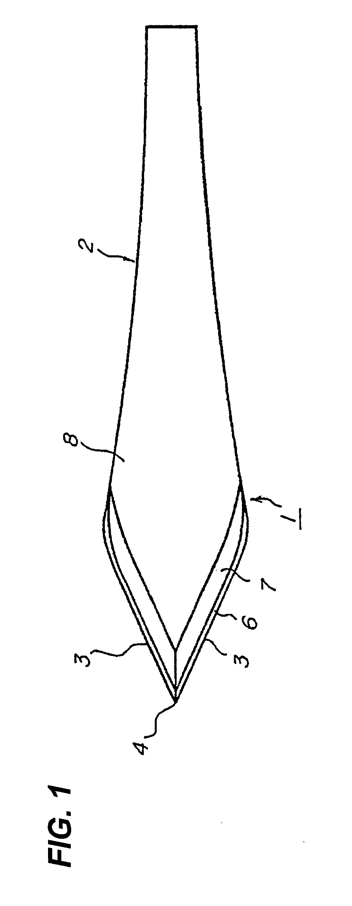

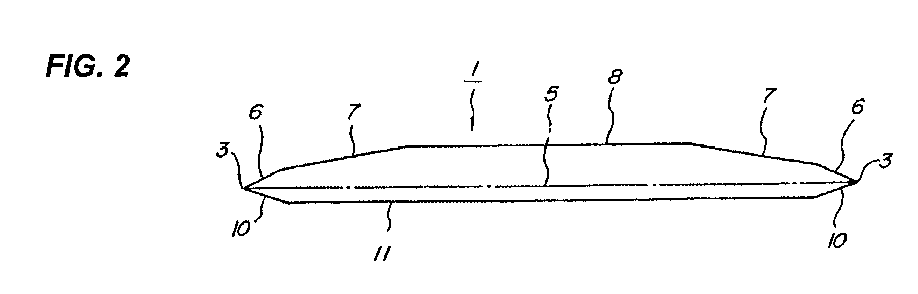

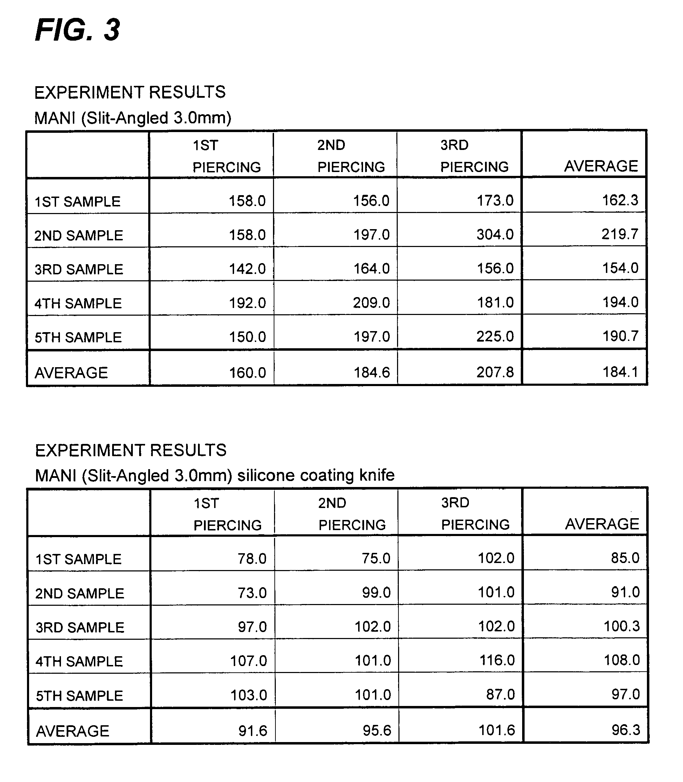

A medical bladed device according to the present invention will be described below with reference to a case where the medical bladed device is applied to an ophthalmic knife used for incising a cornea or a sclera constituting an eyeball. FIG. 1 is a plan view for explaining the shape of a knife according to the present embodiment. FIG. 2 is a sectional view of the knife. FIG. 3 shows tables showing results of a comparative experiment in which the piercing resistance acting when a pig sclera was pierced was measured.

First, the shape of a knife A will be described with reference to FIGS. 1 and 2. In FIGS. 1 and 2, the knife A has a cutting portion 1 and a shank 2 that is formed continuously from the cutting portion 1. A tip of the cutting portion 1 is formed as a sharp pointed tip 4, and an edge 3 serving as a cutting portion is formed along an outer edge of the cutting portion 1 from the pointed tip 4.

A plurality of faces are formed respectively above and below a line 5 connecting the edge 3 formed along the outer edge of the cutting portion 1. Specifically, slanting surfaces 6 and 7 and a flat surface 8 are formed above the edge 3 and a slanting surface 10 and a flat surface 11 are formed below the edge 3. The slanting surfaces 6 and 7 above the edge 3 and the slanting surface 10 and the flat surface 11 below the edge 3 are formed to be smooth surfaces.

A silicone coating layer is formed on the cutting portion 1 of the knife A including the edge 3. The thickness of the silicone coating layer is in a range of 25 nm or more and 5 µm or less. The thickness of the silicone coating layer can be appropriately set by selecting the coating method as will be described later.

The area to form the silicone coating layer is on the cutting portion 1 including the edge 3, but the silicone coating layer need not be formed on the shank 2. However, it does not mean that the silicone coating layer must not be formed on the shank 2 in the course of the coating process.

If the thickness of the silicone coating layer formed on the cutting portion 1 including the edge 3 is less than 25 nm, it is difficult to reduce the piercing resistance when incising a sclera. On the other hand, even if the thickness of the silicone coating layer increases, the piercing resistance does not lower unlimitedly but becomes substantially constant when the thickness of the silicone coating layer is 5 µm or more. Therefore, it is sufficient that the thickness of the silicone coating layer is in the range of 25 nm or more and 5 µm or less.

The inventors of the present application conducted an experiment for comparing the piercing resistance between the knife A having a silicone coating layer of about 1 µm in thickness and a knife without a silicone coating layer. In this experiment, a sclera having a thickness of 0.35 mm or more and 0.40 mm or less was excised from a pig eyeball, piercing was performed through one sclera section once and the piercing resistance during the piercing was measured.

Five samples for each of the knives tobe used in the experiment were prepared to have the same specification including the shape and the size. Piercing through the sclera section was performed three times for each of the samples, and average values thereof were compared to determine the superiority. The results are shown in FIG. 3.

As shown in FIG. 3, in the case of the knife that was not subjected to silicone coating, the average value of the first piercing was 160. 0 mN (millinewton; the same applies hereinafter), the average value of the second piercing was 184.6 mN, and the average value of the third piercing was 207.8 mN. Thus, the average value of the first to third piercings was 184.1 mN. Accordingly, it is found that the piercing resistance increases as the number of times of piercing increases from the first piercing.

On the other hand, in the case of the knife A that was subj ected to silicone coating, the average value of the first piercing was 91.6 mN, the average value of the second piercing was 95.6 mN, and the average value of the third piercing was 101.6 mN. Thus, the average value of the first to third piercings was 96.3 mN. The piercing resistance increases as the number of times of piercing increases from the first piercing also for the knife A.

However, when the piercing resistance of the knife A was compared with that of the knife that was not subjected to silicone coating, the difference was 68.4 mN for the first piercing, 89.0 mN for the second piercing and 106.2 mN for the third piercing. Thus, the difference was 87.8 mN for the average value of the first to third piercings. Accordingly, the piercing resistance was able to be reduced by about a half by applying silicone coating.

Therefore, it is possible to reduce the piercing resistance by applying silicone coating to form a silicone coating layer on the cutting portion 1 including the edge 3.

The inventors of the present application continued measuring the change in the piercing resistance with the change in the thickness of the silicone coating layer. As a result, it is found that the piercing resistance decreases as the thickness of the silicone coating layer increases but the piercing resistance becomes a substantially constant value with little change when the thickness exceeds about 5 µm instead of decreasing at a constant rate. It is also found that the piercing resistance varies widely when the thickness of the silicone coating layer becomes less than 25 nm.

Therefore, it is sufficient that the thickness of the silicone coating layer is 5 µm at a maximum, and the silicone solution may be wastefully used if the thickness is larger. On the other hand, it is difficult to obtain a stable effect if the thickness of the silicone coating layer is less than 25 nm. This is considered to be due to the fact that it is difficult to form the silicone coating layer in a stable thickness.

In view of the above, the piercing resistance can be sufficiently reduced by applying silicone coating in a thickness of 25 nm or more and 5 µm or less to the cutting portion of the medical bladed device (knife A) including the edge.

Even within the thickness range described above, it is also possible to appropriately adjust the thickness of the silicone coating according to the number of times of piercing required for the intended medical bladed device. For example, it is sufficient that the thickness of the silicone coating is 25 nm or more and 1 µm or less if the required number of times of piercing is one. Further, it is sufficient that the thickness is 30 nm or more and 2 µm or less for piercing three times, 40 nm or more and 3 µm or less for piercing five times, and further 50 nm or more and 5 µm for piercing ten times. In general, the piercing is often conducted about three times in a surgical operation using an ophthalmic knife and about ten to fifteen times in a suturing operation using an ophthalmic suture needle.

### Second Embodiment

Next, an embodiment of a coating method will be described. The coating method according to the present invention allows a silicone coating layer having a uniform thickness and free of drop-like lumps to be formed on a cutting portion of a medical bladed device (knife A) including the edge.

The inventors of the present application found that a uniform silicone coating layer can be formed on the surface of the knife A by limiting a raising rate after immersing the knife A in a silicone solution, that the raising rate is preferably low, and that lumps are likely to be formed when the raising rate is higher.

The reason for these is considered to relate to the length of time during which a bridge-like continuous portion is formed between the tip of the knife and the solution surface of the silicone solution due to the surface tension while the silicone solution adhered to the surface of the knife immersed in the silicone solution flows back to the container thereof as the knife A is raised.

Specifically, if the rate of raising the knife A from the solution surface of the silicone solution is high, the time during which the bridge-like continuous portion is formed is short, resulting in that the silicone solution adhered to the surface of the knife A can flow nowhere and remains on the surface, thereby forming drop-like lumps according to the posture of the knife A.

On the other hand, if the raising rate of the knife A is low, the time during which the bridge-like continuous portion is formed is long and a sufficient time can be secured for the silicone solution adhered to the surface of the knife A to flow back into the container via the solution surface.

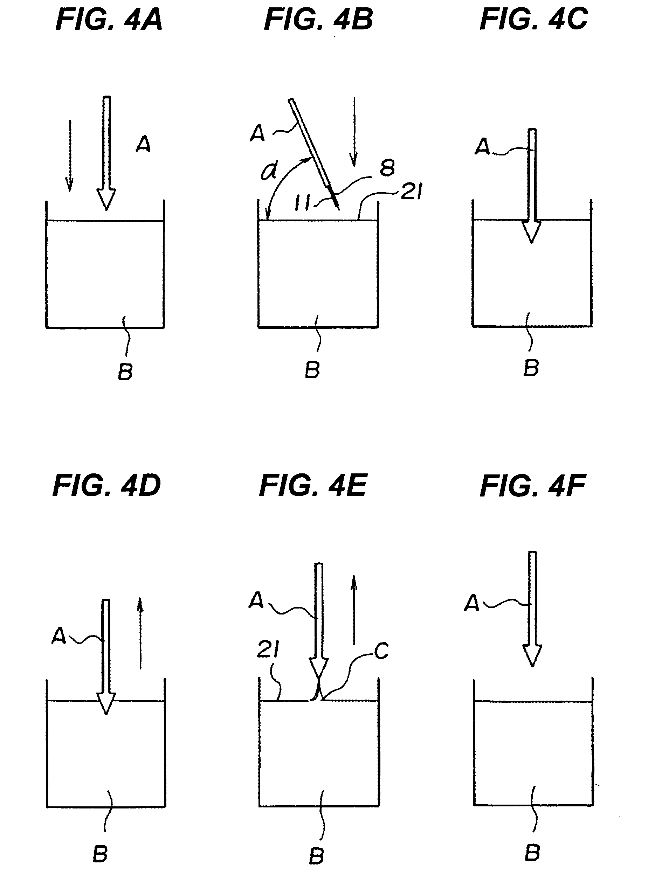

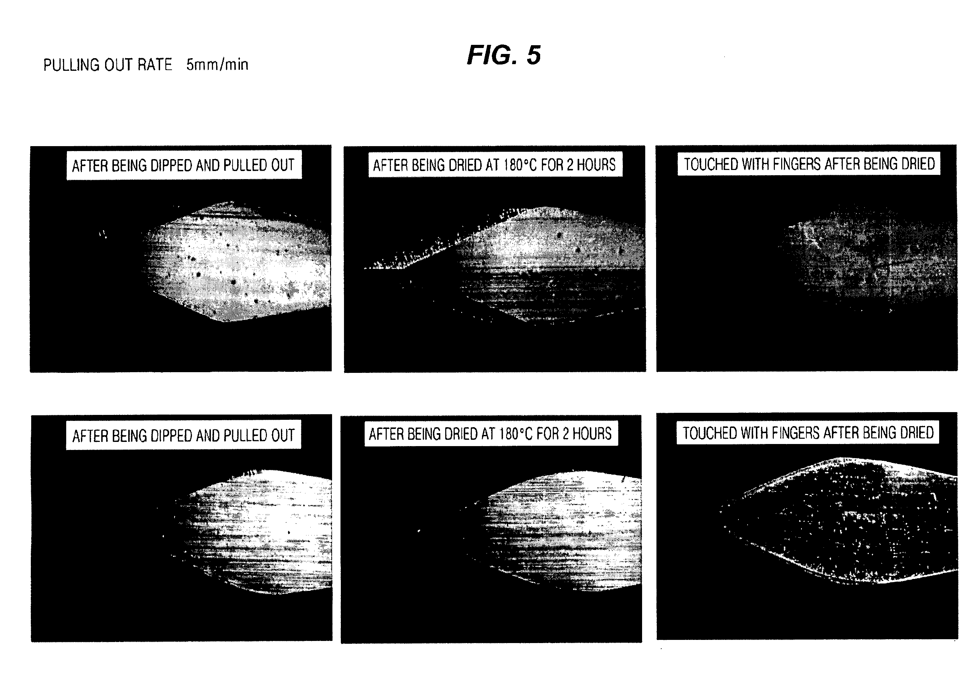

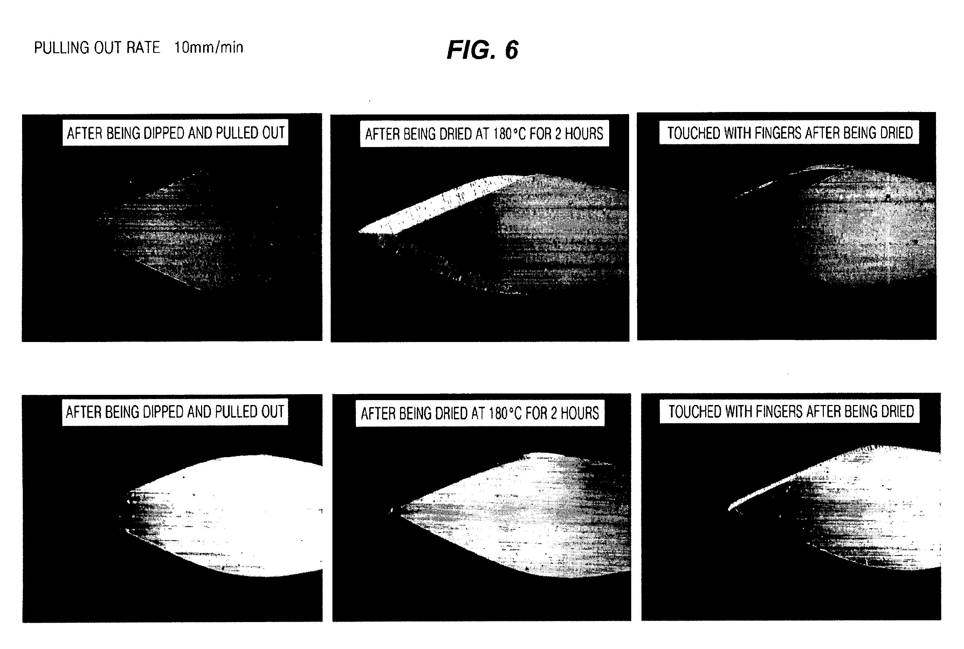

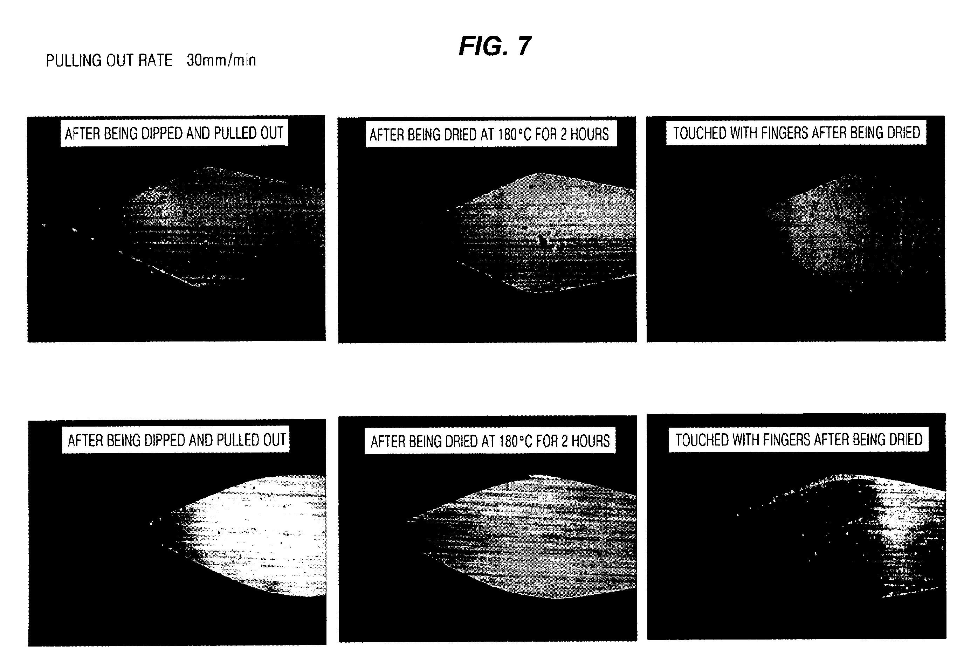

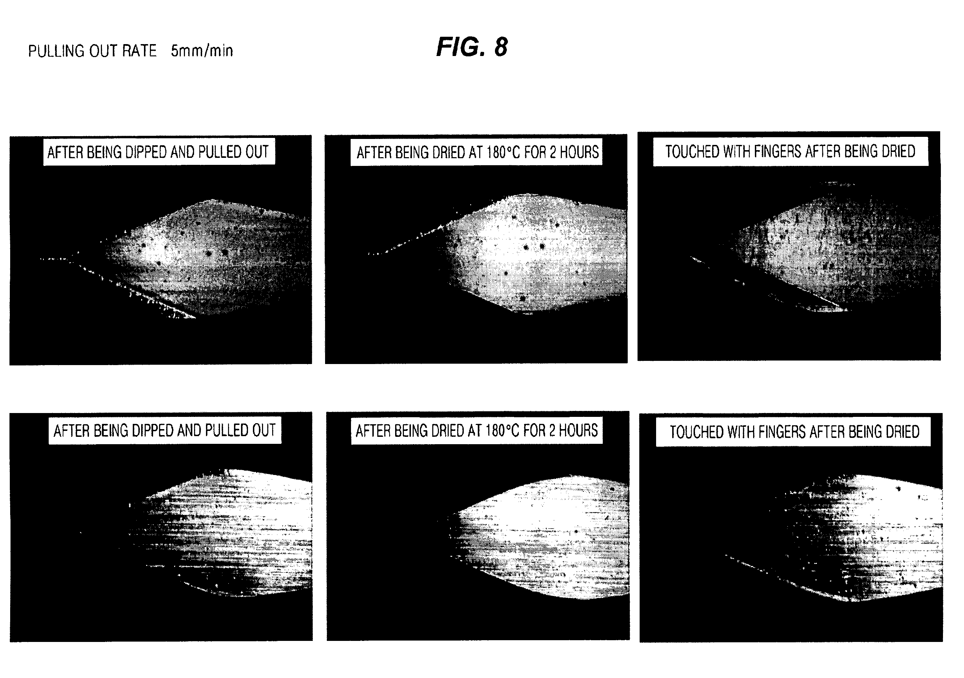

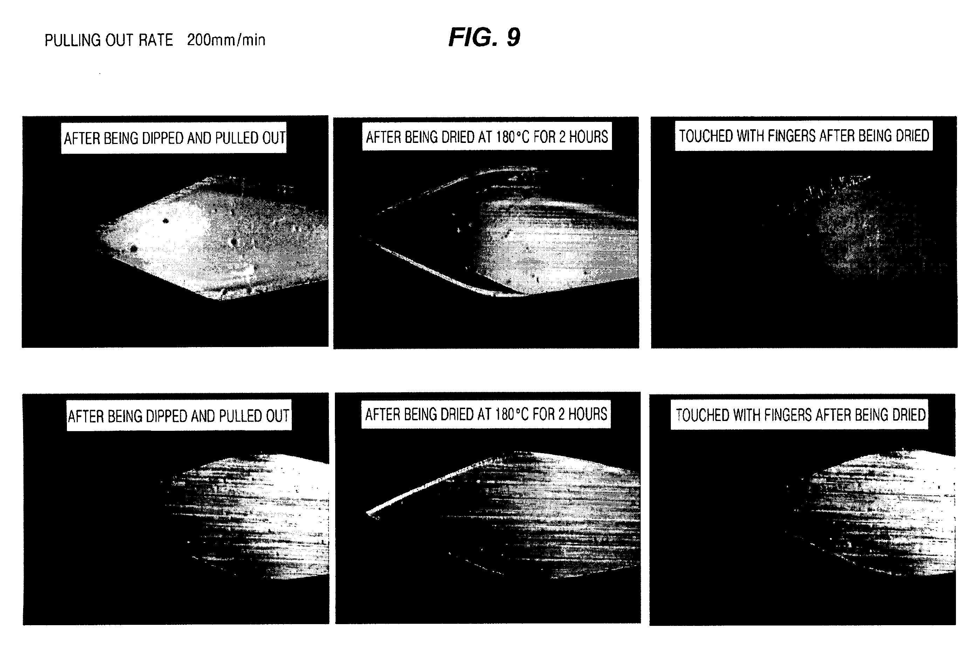

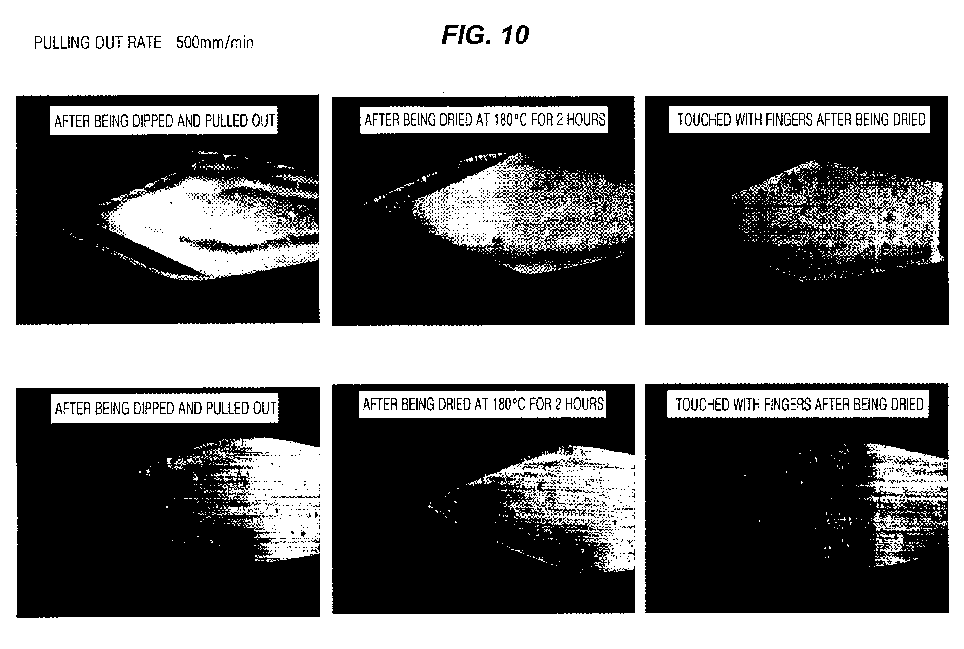

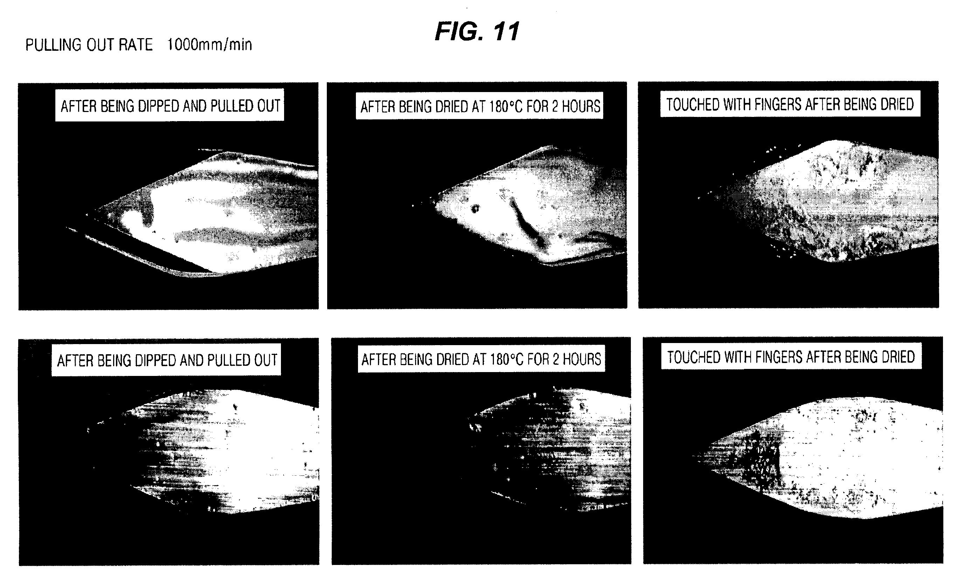

The coating method as described above will now be described with reference to the drawings. FIG. 4 shows schematic views for explaining the coating method. FIG. 5 shows pictures of the surfaces when the knife was raised from the silicone solution at a rate of 5 mmper minute. FIG. 6 shows pictures of the surfaces when the knife was raised from the silicone solution at a rate of 10 mm per minute. FIG. 7 shows pictures of the surfaces when the knife was raised from the silicone solution at a rate of 30 mm per minute. FIG. 8 shows pictures of the surfaces when the knife was raised from the silicone solution at a rate of 50 mm per minute. FIG. 9 shows pictures of the surfaces when the knife was raised from the silicone solution at a rate of 200 mm per minute. FIG. 10 shows pictures of the surfaces when the knife was raised from the silicone solution at a rate of 500 mm per minute. FIG. 11 shows pictures of the surfaces when the knife was raised from the silicone solution at a rate of 1,000 mm per minute.

The container B shown in FIG. 4 contains a silicone solution of a predetermined concentration, in which the knife A is immersed to apply silicone coating thereto. Silicone contained in the silicone solution is not particularly limited. In the present embodiment, a silicone of product number 111343 manufactured by Bluestar Silicones is employed.

As shown in FIG. 4 (a) , the knifeAis placed above the container B and moved down. In this case, means for holding the knife A is not limited and may be any chuck structured to accurately control the raising rate. The knife A is held by the chuck one by one and moved up and down so as to be immersed in and raised from the silicone solution.
The structure is not limited to that for moving the knife A up and down by holding the knife A one by one, but may be a structure for moving the knives A up and down by holding a plurality of knives A at a time with a jig or the like.

When the knife A is immersed in the silicone solution, it is sufficient that the angle α between an upper surface (flat surface 8) or a lower surface (flat surface 11) of the knife A and the solution surface 21 of the silicone solution shown in FIG. 4 (b) is 0° or larger and 90° or smaller. This range of the angle is obtained as a result of the experiment by the inventors of the present application.

Next, as shown in FIG. 4(c), the knife A is moved down in the direction of the arrow to immerse the cutting portion 1 including the edge 3 into the silicone solution. At this time, the lowering rate of the knife A is not limited and may be any rate. In addition, the part which is immersed in the silicone solution needs to include at least the cutting portion 1 including the edge 3, and it is not a problem even if a part closer to the shank 2 is also immersed.

After the knife A is immersed in the silicone solution, the knife A is raised in the direction of the arrow to be taken out of the silicone solution as shown in FIG. 4 (d) . At this time, the raising rate of the knife A is set in the range of 500 mm per minute or lower.

Specifically, as shown in FIG. 4 (e) , when the pointed tip 4 of the knife A is pulled out from the solution surface 21 of the silicone solution, a bridge C is formed between the pointed tip 4 and the solution surface 21 due to the surface tension of the silicone solution. It is to be considered that the silicone solution adhered to the surface of the knife A drops into the container B through the bridge C. Accordingly, as the duration of the bridge C is longer, excess silicone solution is less likely to remain on the surface of the knife A and thus a smoother and more uniform silicone coating layer can be formed. Therefore, the lower limit of the raising rate of the knife A does not need to be particularly specified. However, in terms of productivity when it is assumed that silicone coating is performed for several tens to several thousands of knives A at a time, a rate of 0.5 mm per minute or higher is preferable. For more efficient production, the silicon coating is preferably performed at the raising rate of about 5 mm per minute or higher and 200 mm or lower.

Then, as shown in FIG. 4 ( f ) , the knife A is dried after being completely pulled out from the silicone solution. In this manner, it is possible to produce the knife Aapplied with silicone coating on the cutting portion 1 including the edge 3.

The drying of the knife Apulled out from the silicone solution can also be performed by leaving the knife A in the air so that the knife A is dried naturally. However, the knife A is preferably retained within a predetermined temperature range for a predetermined time to retain the quality of the silicone coating layer. Therefore, in the present embodiment, the knife Apulled out from the silicone solution is exposed in an atmosphere at a temperature range of 100°C or higher and 250°C or lower for 30 minutes or more for forced drying.

Next, the states of the surfaces of the knife A when the raising rate of the knife A immersed in the silicone solution is varied will be described with reference to FIGS. 5 to 11. A silicone solution having the silicone concentration of 10% is used in the experiments.

For example, if the silicone solution has a higher concentration, the viscosity becomes higher, drop-like lumps is more likely to be produced on the surface of the knife A and the surface becomes less easily dried. In the experiments conducted by the inventors of the present application, it was found that the drop-like lumps were less likely to be formed and the surface dried easily with the silicone solution having the concentration in the range of 0.2% by weight or higher and 15% by weight or lower. In particular, the drop-like lumps were less likely to be formed and the surface dried easily with the silicone solution having the concentration in a range of 2% by weight or higher and 15% by weight or lower when the immersion into the silicone solution and the drying are performed once, and with the silicone solution having the concentration in a range of 0.2% by weight or higher and 10% by weight or lower when the immersion into the silicone solution and the drying are repeated a plurality of times.

FIG. 5 shows pictures of the surfaces (upper and lower surfaces) when the knife A was raised at a rate of 5 mm per minute. As is clear from FIG. 5, drop-like lumps were not formed on the surfaces, and the surfaces were not affected even when they were touched by a hand after being dried at 180°C for 2 hours. That is, the surfaces were sufficiently dried under the aforementioned drying condition.

FIG. 6 shows pictures of the surfaces (upper and lower surfaces) when the knife A was raised at a rate of 10 mm per minute. As is clear from FIG. 6, drop-like lumps were not formed on the surfaces, and the surfaces were not affected even when they were touched by a hand after being dried at 180°C for 2 hours. That is, the surfaces were sufficiently dried under the aforementioned drying condition.

FIG. 7 shows pictures of the surfaces (upper and lower surfaces) when the knife A was raised at a rate of 30 mm per minute. As is clear from FIG. 7, drop-like lumps were not formed on the surfaces, and the surfaces were not affected even when they were touched by a hand after being dried at 180°C for 2 hours. That is, the surfaces were sufficiently dried under the aforementioned drying condition.

FIG. 8 shows pictures of the surfaces (upper and lower surfaces) when the knife A was raised at a rate of 50 mm per minute. As is clear from FIG. 8, drop-like lumps were not formed on the surfaces, and the surfaces were not affected even when they were touched by a hand after being dried at 180°C for 2 hours. That is, the surfaces were sufficiently dried under the aforementioned drying condition.

FIG. 9 shows pictures of the surfaces (upper and lower surfaces) when the knife A was raised at a rate of 200 mm per minute. As is clear from FIG. 9, drop-like lumps were not formed on the surfaces, and the surfaces were not affected even when they were touched by a hand after being dried at 180°C for 2 hours. That is, the surfaces were sufficiently dried under the aforementioned drying condition.

FIG. 10 shows pictures of the surfaces (upper and lower surfaces) when the knife A was raised at a rate of 500 mm per minute. As is clear from FIG. 10, drop-like lumps were not formed on the surfaces but slight bulges of silicone were formed along the flat surface 8. Then, the surfaces were affected in a manner that the silicone was slightly peeled off when they were touched by a hand after being dried at 180°C for 2 hours.

FIG. 11 shows pictures of the surfaces (upper and lower surfaces) when the knife A was raised at a rate of 1,000 mm per minute. As is clear from FIG. 11, drop-like lumps were formed on the surfaces, and the surfaces were affected in a manner that the silicone was peeled off when they were touched by a hand after being dried at 180°C for 2 hours.

As described above, the rate at which the knife A is raised from the silicone solution is limited to 500 mm per minute at the highest, and uniform silicone coating can be applied without forming drop-like lumps on the surfaces of the knife A by raising the knife A at a rate lower than 500 mm per minute.

Although a method of immersing the knife A into the silicone solution only once for coating is employed in the aforementioned embodiment, it is of course also possible to apply silicone coating to the knife A a plurality of times by the aforementioned method to thereby appropriately adjust the thickness. In addition, although the drying is performed under the temperature condition of 180°C and the condition of 2 hours in the present embodiment, the conditions are not limited thereto. As a result of the experiments by the inventors of the present application, a desired state of the surfaces of the knife A was obtained by drying at a temperature range of 100°C or higher and 250°C or lower for 30 minutes or more.

### Industrial Applicability

The medical bladed device according to the present invention can be advantageously used in such a case as piercing one affected site once or a plurality of times.

In addition, the coating method according to the present invention can apply uniform silicone coating on the surfaces of a medical bladed device and can be advantageously used in coating of a knife, a scalpel or a suture needle.

## Claims

1. A medical bladed device comprising:
a cutting portion having an edge on an outer side thereof; and
a shank that is continuous from the cutting portion,
wherein the cutting portion including the edge is coated with silicone in a thickness of 25 nanometers or larger and 5 micrometers or smaller.

2. A method of coating a medical bladed device having a cutting portion and a shank that is continuous from the cutting portion by immersing the medical bladed device into a silicone solution to apply silicone coating on a surface of the cutting portion, the method comprising:
immersing the cutting portion into the silicone solution while keeping the cutting portion side down and the shank side up and an angle between an upper surface or a lower surface of the cutting portion and a solution surface of the silicone solution within a range of 0 degree or larger and 90 degrees or lower; thereafter
raising the medical bladed device at a rate of 500 mm per minute or lower within the angle range to pull the cutting portion out from the silicone solution; and
drying at a temperature range of 100°C or higher and 250°C or lower for 30 minutes or more.

3. The method of coating a medical bladed device according to claim 2, wherein a thickness of a silicone film is adjusted by setting a silicone concentration to be 0.2% by weight or higher and 15% by weight or lower and by performing the immersion into silicone and the drying once or a plurality of times repeatedly.
